# EUROPEAN PATENT APPLICATION

(11) **EP 2 264 242 A1**
(43) Date of publication of application: **22.12.2010**
(21) Application number: 09162790.1
(22) Date of filing: 16.06.2009
(51) Int. Cl.: D06M 23/00, D06M 23/02, A47L 13/17, A61K 8/02

(54) **Nonwoven fabric products with enhanced transfer properties**

(71) Applicant: Ahlstrom Corporation, 00180 Helsinki (FI)
(72) Inventor: Pedoja, Roberto, FI-00180, Helsinki (FI)
(74) Representative: Hovi, Simo Pekka Tapani

(57) **Abstract**

The present invention relates to the field of non-woven textile products, and is applied to the manufacture of nonwoven fabric products or generally nonwovens for various applications such as household use and personal care.

In particular, the present invention relates to a wet nonwoven fabric product impregnated with a substance for personal care, household application or topic medical use, **characterised in that** the said nonwoven fabric is a bonded web structure with hydrophilic properties or that is made hydrophilic by treatment with wetting additives.

## Description

The present invention relates to the field of nonwoven textile products and is applied to the manufacture of nonwoven fabric, particularly general nonwoven products, for various applications such as household use and personal care.

Wet or impregnated wipes are of wide application in several fields. For example, nonwoven fabric cloths are used for cleaning purposes and may be impregnated with waxes or other cleaning solutions. Wet wipes are also used for personal care and may contain detergents, perfumes or even cosmetic lotions or creams.

These products are normally made from cellulose-based raw materials (100% cellulose or in any case a high cellulose content), such as viscose, cotton and the like, which are provided with absorbent properties.

High absorbent properties are necessary for the cloth to be wetted and retain a sufficiently high amount of the substance solution of the desired type (detergent, cosmetic and so on). However, another important property for such products is the capacity to release such a substance solution and to transfer it to the surface to be treated. The prior art cellulose-based cloths as described above do not fulfil this requisite, as the substance solution is strongly retained by the cellulose fibres so that only 50-60% of the substance solution is released during application.

The consequence of this partial release of the substance solution is not only a poor efficiency of the product and an increase of the costs for the user, but also the fact that the resulting waste cloths are still impregnated with potentially polluting substances.

An object of the present invention is therefore to provide a wet nonwoven fabric product impregnated with a substance endowed with high absorbent properties and increased release properties of the said substance to a surface.

A further object of the invention is the process for preparing said textile product.

These objects have been achieved by a wet nonwoven fabric product impregnated with a substance, **characterised in that** the said nonwoven fabric is a bonded web structure with hydrophilic properties or that is made hydrophilic by treatment with wetting additives, and by a process for manufacturing the said product, as defined in the appended claims, whose definitions are integral part of the present description.

The nonwoven fabric material to be used in the present invention may comprise fibres or continuous filaments or a mixture thereof.

The continuous filament may be obtained by a spinning process by means of 1- to 5-orifices, preferably 2-3 orifices, spinner.

The continuous filaments may be produced as monocomponent filaments, or as bicomponent, tricomponent or multi-component filaments.

In one embodiment, the fibres comprise more than about 90% of synthetic fibres, more preferably more than 95% of synthetic fibres. In a particularly preferred embodiment, the fibres used for the manufacturing of the web comprise essentially 100% and preferably 100% of synthetic fibres.

In one embodiment, the continuous filaments comprise more than about 90% of synthetic continuous filaments, more preferably more than 95% of synthetic continuous filaments. In a particularly preferred embodiment, the continuus filaments used for the manufacturing of the web comprise essentially 100% and preferably 100% of synthetic continuous filaments.

In one embodiment, the synthetic fibres (staple fibres) and/or continuous filaments are made of a material selected from the group consisting of polyester, polypropylene, polyamide, acrylic and mixtures thereof; preferably they are 100% polyester or 100% polypropylene.

In one embodiment, the synthetic fibres and/or continuous filaments are made of a material selected from the group consisting of polyester, polypropylene, polyamide, acrylic and mixtures thereof, preferably they are 100% polyester or 100% polypropylene, and contains from 5 to 3 0 w % of bicomponent polyester/polyethylene, polypropylene/polyester, PET polyester/PEN polyester, Nylon-6,6/PCT polyester, Nylon-6 co-polyamide, Polylactic acid/polystyrene, acetal polyurethane or soluble copolyester HDPE/LLDPE fibres.

The term "bicomponent fibre" means a fibre obtained by co-extrusion of two different polymers. They are also called "conjugate fibres". Non-limiting examples of bicomponent filament configurations include sheath-core (concentric or eccentric), side-by-side (equal or unequal portions), and islands-in-a-sea. For the inventive application, sheath-core bicomponent fibres and side-by-side fibres are especially suitable.

In one embodiment, HOLLOFIL^{®} fibres are used, alone or in admixture with the above fibres or continuous filaments. HOLLOFIL^{®} is a trademark of DuPont that indicates hollow fibres made of silicone-treated 100% polyester (Dacron, polyethyleneterephtalate).

In one embodiment, the inventive nonwoven fabric material is a bonded web structure that comprises more than one carded web, preferably 3 to 9 carded webs. With a number of webs in the range specified above, a more isotropic textile structure, and accordingly a maximized spatial layout of the fibres is achieved, which results into a maximized fibre-water contact surface. Thereby, the water droplets are adsorbed by the structure within the small spaces resulting from the random distribution of the fibres.

The textile product according to the invention typically has a weight/surface ratio (basis weight) ranging between 20 and 150 g/m², preferably 20-50 g/m² or more than 25 and less than 35 g/m².

The bonded web structure according to the invention can be obtained according to various processes, known to the skilled artisan, such as the ones described in the following embodiments.

In a first embodiment, the inventive web structure can be bonded by a well-known hydroentanglement process, thus obtaining a so called spunlace web structure. This process provides for entanglement of the web fibres, moving on a perforated or patterned screen, by means of high pressurized water jets. The water pressure generally increases from the first to the last injectors, pressures up to 2200 psi are used to direct the water jets onto the web. The impinging of the water jets on the web causes the entanglement of the fibres. Exhaust water is removed by vacuum from the screen, to avoid flooding of the web. The free water being entrapped among the fibres is then eliminated by means of drying.

Optionally, the hydroentanglement process can be followed by a hydroembossing treatment according to conventional techniques. This allows to improve the softness of the product and to increase its volume.

In another embodiment, bonding of the web structure is accomplished by means of a so called dry-laid process. Dry-laying procedure may comprise providing a web, normally a carded web, as defined above comprising 5 to 30 w% of a bicomponent fibre as defined above, and passing it into a dryer wherein the material is subject to temperatures of between 120 and 200°C for a time generally comprised between 3 and 15 seconds. The high temperature melts the low-temperature melting polymer of the bicomponent fibres, thus establishing bonding points throughout the web. This process, known as air through bonding, is advantageous if a softer and thicker web structure is desired, especially if a side-by-side bicomponent fibre is used. It should be noted that such softer structure improves the adsorbing and releasing properties of the material, that are important for the purposes of the invention.

In a still different embodiment of the invention, the inventive web structure is bonded by a well-known chemical bonding process. In this case, the web structure is treated - for example by impregnation, printing, spraying, powder or foam application - with a solution or a solid form of a latex polymer or a binder, in amounts ranging from 5 to 60 w%. The so treated web structure is then cured, for example by heat treatment. Suitable binders are selected from styrene-butadiene rubber, vinyl copolymers, vinyl acetate, styrenated or vinyl acrylates, polyvinylchloride. The binder formulation may comprise further ingredients, such as: surfactants (to improve binder adhesion, stability and ability to be converted into a foam); external crosslinkers; defoamers (to minimize foam in the process); repellent agents; salts (to impart low flame response properties and to convey antistatic properties); thickeners (to control rheology of the binder liquid); catalysts (to promote curing and cross-linking); acids and bases (to control pH of the binder); dyes and pigments; fillers (to reduce binder tack and to lower cost); optical brighteners (to increase whiteness); sewing aids (to provide lubrication).

In another embodiment, bonding of the web structure is accomplished by means of a needle-punching process. A web is provided, normally a carded web of staple fibres. The web is mechanically entangled by means of a multiplicity of barbed metal needles which mechanically move in a rapid reciprocating fashion forwards and backwards through the web, in a direction essentially perpendicular to the plane of the web. This movement of the barbed needles causes fibres within the web to become entangled with nearby fibres, making the web a coherent, strong structure.

As said above, the product of the invention comprises a bonded web structure with hydrophilic properties (inside the polymer) or that is made hydrophilic by treatment with wetting additives.

A web structure with hydrophilic properties can be a web structure that was subject to a surface hydrophilic finishing treatment. These nonwovens are available on the market. However, in this case hydroentanglement is not suitable to create a bonded structure, as the water jets treatment removes the hydrophilic finish. Therefore, if such material is used, dry-laying or chemical bonding are preferred.

A web structure that is made hydrophilic by treatment with wetting additives can be obtained as described below.

The wetting treatment can be carried out both on previously existing spunlace product or other bonded web structures, and upon manufacture of the same.

If hydroentanglement is used to bond the web structure, the wetting treatment is carried out after hydro-entanglement and before oven-drying.

The wetting additives are those commonly employed in the field, such as cationic, anionic or non-ionic surfactants.

In a preferred embodiment, an anionic surfactant is used as a wetting agent. The said anionic surfactant is preferably an alkyl-polyglucoside ester. In one embodiment, the said alkyl polyglucoside ester is selected from disodium cocopolyglucose sulphosuccinate, disodium cocopolyglucose citrate, sodium cocopolyglucose tartrate or mixtures thereof. These surfactants are known with the trademark EUCAROL® AGE SS, EUCAROL® AGE EC and EUCAROL® AGE ET, respectively, and are commercially available from LAMBERTI spa. In the course of testing, this additive exhibited a great efficacy in terms of giving absorption capacity to the final product with a low dosage. In fact, the low dosage addition of additives leads to improved quality products as compared with those products obtained with high dosages, as will be discussed in detail below.

The wetting treatment method can be selected from those used in common practice. They are, *inter alia*:
- Spraying: the product mixture will be sprayed on the fibres;
- coating: the product mixture will be applied by a contact roller spreading the products on the web surface;
- printing: the same as coating, but using a printing machine;
- impregnation; the web structure, either already built up or in an intermediate step, is dipped in a mixture of selected products, then the excess amount is wrung by means of a mangle or similar systems;
- foam coating: a mixture of selected products is mixed with a gas (usually air) in a high speed agitator until its physical state is that of a foam, and the foam is then applied to the web by conventional coating techniques.

The dosage of the wetting additive changes as a function of the fibres being used, the nonwoven features (density, fibre distribution, etc.) and desired level of adsorption (meaning the amount of adsorbed, retained water and the adsorption time, capillarity).

Impregnation followed by a squeezing mangle is the preferred treatment method, which is advantageous over the other methods in that it allows the wetting additive to be spread more evenly throughout the fibre portions, even inside the nonwoven. In this case, the bath concentration will range between 1 and 10 g/l, preferably 1-5 g/l. For example, if a solution with 1 g/l additive concentration is used, with the nonwoven fabric absorption being 100%, a 1% amount of absorbed additive will be obtained.

In this case, the web structure is passed into a wetting additive bath at the concentrations described above, then sent to the squeezing mangle and subsequently to the oven for drying.

The squeezing mangle is to be set such that the squeezing is calibrated, i.e. the pressure should be such that a very small amount of liquid is left, in order to make the stay in the drying oven as short as possible. At the same time, the pressure should not be as high as to calender the web structure, which would result in modifying the nonwoven fabric architecture. After a number of tests, it has been found that the best results are obtained with pressure values ranging between 4 and 8 bars, preferably around 6 bars, when applied to a web structure dipped in a wetting additive solution at the above concentrations.

Following the squeezing mangle, the nonwoven fabric is sent to the drying oven. The temperature within the oven depends on the speed at which the product passes there through and is to be adjusted such that complete evaporation of the water is ensured. For example, if a product with 55 g/m² grammage is produced at 150 m/min, the temperature within the oven is to be set at about 120°C for complete water evaporation.

The inventive web structure may contain various additives. After a number of tests, it has been found that the quality of the final product, particularly in terms of softness and absorption capacity, depends on the percentage of additives provided on the starting fibres.

Several types of additives are added to the synthetic fibres or continuous filaments by the manufacturers, such as: lubricating additives to give smoothness and easy processability; antistatic additives to prevent damaging electrostatic currents that may degrade the product, or at worst, reduce the productivity of the machine; hydrophilic additives; anti-foam additives to avoid that foam may be generated, especially upon the hydro-entanglement step.

The synthetic fibres or continuous filaments that can be used for the purposes of the present invention should have a very low percentage of starting additives. For example, a percentage ranging from 0,1 w% to 0,2 w% is suitable. Examples of these fibres are 100% polyester fibres purchased from Dupont, Montefibre, Hochst, Catalana, etc..

In one embodiment, the inventive nonwoven fabric, in addition of being treated with wetting additives, is also treated with anti-foam additives in order to adjust the surface tension of the wetting agent solution. Examples of said anti-foam agents are silicone-based additives. The preferred anti-foam agent is HANSA SP® available from HANSA spa. The anti-foam agent can be applied to the nonwoven fabric using the various treatment methods as described above. Advantageously, the anti-foam agent is applied by impregnation, simultaneously with the wetting agent, i.e. one single bath is prepared containing both the wetting agent and the anti-foam additive. The anti-foam agent solution that is advantageously employed for the inventive product has a concentration ranging between 1 and 7 g/l, preferably about 2 g/l.

As said above, the anti-foam agent serves to adjust the surface tension of the wetting agent, i.e. the capacity of generating foam upon application. Hydrophilic additives are also available, which are little effective though being poorly foam-forming, and hence require to be used at high dosages. As already discussed above, both the starting additives and the hydrophilic and anti-foam additives that are added upon manufacture impair the final quality of the product when they are added at high dosages. Therefore, the best choice is that suggested by the present invention, i.e. using fibres with low amounts of starting additives, to which reduced amounts of very foam-forming wetting agent, particularly alkyl-polyglucoside esters such as described above, and simultaneously also reduced amounts of an anti-foam additive are applied.

As said above, the web nonwoven fabric product of the invention is obtained by impregnating with a substance the said bonded web structure having hydrophilic properties or that is made hydrophilic by treatment with wetting additives as described before.

The term "substance" as used herein means a solution, suspension, gel, emulsion or other wet formulation of a substance for personal care, household application or topic medical use or mixtures thereof, as the case may be.

Non-limiting examples of a substance for personal care are: solutions or lotions for personal hygiene and/or sanitization, skin creams, lotions or waxes, tanning creams or lotions, sunscreen formulations, insect repellent formulations, deodorants, perfumes, antibacterial, antiviral and/or antifungal formulations, make-up removing lotions or solutions and cosmetic products in general.

Non-limiting examples of a substance for household application are: detergent solutions or emulsions, waxes for ceramic or wooden floor, waxes for wood furniture surfaces, surface disinfectants, antibacterial, antiviral and/or antifungal products for household use, metal polishing emulsions, solutions or creams and cleaning formulations in general.

Non-limiting examples of a substance for topic medical use are: skin disinfectants, skin antibacterial, antiviral and/or antifungal substances, cicatrizing formulations and in general any drug formulation that can be administered by topical application, or sanitizing compositions for medical facilities, appliances or devices.

In particular, when the substance is a viscous substance as may be the case with emulsions, suspensions, gels, creams or waxes, it is important that the wetting agent is particularly efficient to impart hydrophilicity to the nonwoven fabric and to favor the subsequent release of the substance during use. In one embodiment, with these viscous substances a nonwoven treated with an alkyl-polyglucoside ester is advantageously used. More preferably, disodium cocopolyglucose solphosuccinate can be used, due to its fluidifying effect.

The use of a substance in the form of a cream, wax or gel is particularly preferred, as these composition forms contain a lesser amount of water, which is beneficial to delay the growing of undesired microorganism colonies. In this case, minor amounts of biocidal additives can be used or even the product can be substantially free from biocidal additives.

If a cream, wax or gel is used as the substance, it is convenient that this substance is able to lower its viscosity at a temperature of 30-35°C, so as to facilitate its application to a human body.

The inventive web structure can be impregnated with the said substance by any known method as the ones described above for wetting treatment, with the proviso that the impregnated nonwoven fabric product of the invention is packaged while still wet. In particular, delivery of the substance on the web by means of suitable nozzles can advantageously be used. Impregnation as well as spraying are suitable methods for solutions or lotions. For creams and waxes, hot melt delivery can advantageously be used, due to the high viscosity of such substance forms.

The inventive textile product can be for example in the form of cloths, rags and similar fabrics, wipes, etc.

The textile product obtained according to the invention is highly hydrophilic, provided with a considerable adsorbing power and softness, and good hand property, also persisting after first use.

Additionally it has been surprisingly found that, besides this capacity to adsorb water compositions, at the same time it has enhanced releasing properties of such water compositions as pressure is applied, for example if the fabric is squeezed between the fingers or is pressed against a surface to be treated.

It has been experimentally determined that more than 80 vol%, or even more than 90 vol% of the substance is released from the nonwoven fabric product upon pressure application. The term "pressure application" means the normal average pressure that is exercised by a user's hand when a cloth, pad or other piece of fabric is applied to the surface to be treated.

This high released amount of substance is in contrast with the only 50-60 vol% (or even less in case of waxes / creams) of releasing that can be accomplished with the cellulose-based prior art nonwoven fabric products. Without being bound to any theory, this is probably due to the fact that in the fabric of the present invention the fibres or continuous filaments of the web structures do not absorb the substance, which is only retained in the interstices between the fibres or continuous filaments.

The volume ratio between voids and fibres in the inventive fabric is therefore an important feature. Thus, the apparent density of the fabric, defined as the weight per volume unit, wherein the volume is given by the fibres volume and the voids volume, will be preferably between 20 gr/dm³ and 30 gr/dm³.

The parameters that can influence the achievement of this low density are both the diameters of the fibres or filaments and the method used for entangling these fibres or filaments.

The fibres or continuous filaments will preferably have a diameter between 0.5 dtex and 6.7 dtex; preferably a diameter of between 0.9 and 2.5 dtex, more preferably about 2 dtex, characterizes the continuous filaments, while a diameter of between 0.5 and 3.7 dtex, preferably about 3.3 dtex, is preferred for staple fibres.

The preferred entangling method for increasing the volume of the fabric in the present invention is the one that allows to increase the thickness of the fabric of from 20% to 70%, while keeping the basis weight substantially constant. The preferred entanglimg method is air-through bonding. This method has the further advantage to avoid the use of water, thus helping in preserving the final product against microorganism proliferation.

Particularly, the inventive product has a lower adsorption time of the substance, a greater capacity of retaining the same, as well as a greater capacity to release the substance upon pressure application and improved overall softness.

In the course of testing, the cloth prepared as described above exhibited a considerable adsorbing power, which has remained quite unchanged upon use (Table 1).

**Table 1**

| | SAMPLE | DRY | WET | ABSORPTION |
|---|---|---|---|---|
| | | WEIGHT | WEIGHT | |
| | | /g(m²) | /g(m²) | (%) |
| | 100% polyester | | | |
| | Non treated | 51 | 399 | 682 |
| | | | | |
| | Treated | 48 | 411 | 756 |
| 1g/l | | | | |
| | Treated | 47 | 445 | 847 |
| 2g/l | | | | |
| | Treated | 48 | 473 | 885 |
| 5g/l | | | | |

The assessment of the adsorbing power has been carried out in accordance with the EDANA 10.4-02 ABSORPTION standard.

Furthermore, in comparative tests, the inventive product, at the same thickness, has proved to have an improved strength to longitudinal and transverse load and improved longitudinal and transverse elongation properties as compared with a cloth consisting of a 50% viscose and 50% polyester mixture, such as illustrated in Tables 2 and 3.

**Table 2**

| | | Akena P40 | VP 40 |
|---|---|---|---|
| | Thickness (mm) | 0,57 | 0,56 |
| | Load L/T (N) | 70/24 | 46/11,5 |
| | Elongation L/T | 52/160 | 38/137 |
| (%) | | | |

**Table 3**

| | | Akena P50 | VP 50 |
|---|---|---|---|
| | Thickness (mm) | 0,62 | 0,58 |
| | Load L/T (N) | 115/31 | 64/16 |
| | Elongation L/T | 49/155 | 38/135 |
| (%) | | | |

Akena P40 and P50 = 100% polyester inventive cloth having a grammage of 40 g/m² and 50 g/m², respectively.
VP40 and VP50 = comparative cloth of 50% viscose and 50% polyester having a grammage of 40 g/m² and 50 g/m², respectively.
L/T = longitudinal and transverse.

## Claims

1. A wet nonwoven fabric product impregnated with a substance for personal care, household application or topic medical use, **characterised in that** the said nonwoven fabric is a bonded web structure with hydrophilic properties or that is made hydrophilic by treatment with wetting additives.

2. The product according to claim 1, wherein the said bonded web structure has thickness increased of from 20% to 70% with respect to the corresponding non-bonded nonwoven fabric from which it derives.

3. The product according to claim 1 or 2, wherein the said web structure comprises fibres or continuous filaments or a mixture thereof.

4. The product according to claim 3, wherein the fibres comprise more than about 90% of synthetic fibres, or more than 95% of synthetic fibres; or the fibres comprise essentially 100% or 100% of synthetic fibres.

5. The product according to claims 3 or 4, wherein the continuous filaments comprise more than about 90% of synthetic continuous filaments, or more than 95% of synthetic continuous filaments; or the continuous filaments comprise essentially 100% or 100% of synthetic continuous filaments.

6. The product according to claims 3 or 5, wherein the said continuous filaments are produced as filaments with a monocomponent, bicomponent, tricomponent or multicomponent configuration.

7. The product according to any of claims 1 to 6, wherein said synthetic fibres and/or continuous filaments are made of a material selected from the group consisting of polyester, polypropylene, polyamide, acrylic and mixtures thereof, or 100% polyester or 100% polypropylene.

8. The product according to any of claims 1 to 6, wherein said synthetic fibres are hollow fibres made of silicone-treated 100% polyester.

9. The product according to claim 7 or 8, further containing from 5 to 30 w% of bicomponent fibres.

10. The product according to claim 9, wherein said bicomponent fibres are selected from polyester/poliethylene, polypropylene/polyester, PET polyester/PEN polyester, Nylon-6,6/PCT polyester, Nylon-6 co-polyamide, Polylactic acid/polystyrene, acetal polyurethane or soluble copolyester HDPE/LLDPE fibres.

11. The product according to claim 9 or 10, wherein the said bicomponent fibres have a sheath-core structure.

12. The product according to claim 9 or 10, wherein the said bicomponent fibres have a side-by-side structure.

13. The product according to any of claims from 1 to 12, wherein the said nonwoven fabric is a bonded web structure that comprises more than one carded web, preferably 3 to 9 carded webs.

14. The product according to any of claims 1 to 13, having a basis weight ranging between 20 and 150 g/m² or between 20 and 50 g/m² or more than 25 gr/m² and less than 35 gr/m².

15. The product according to any of claims from 1 to 14, wherein the said bonded web structure is a spunlace web structure, optionally a spunlace hydroembossed web structure, that is made hydrophilic by treatment with wetting additives after the hydroentanglement step.

16. The product according to any of claims 9 to 14, wherein the said bonded web structure is a dry-laid or air through bonded web structure.

17. The product according to any of claims from 1 to 16, wherein the said bonded web structure is a chemically bonded web structure further containing 5 to 60 w% of binders or latexes for accomplishing the said chemical bonding step.

18. The product according to any of claims 1 to 17, wherein the said wetting additives are selected among cationic, anionic or non-ionic surfactants.

19. The product according to claim 18, wherein the said wetting additive is an alkyl-polyglucoside ester anionic surfactant.

20. The product according to claim 19, wherein the said alkyl polyglucoside ester is selected from disodium cocopolyglucose sulphosuccinate, disodium cocopolyglucose citrate, sodium cocopolyglucose tartrate or mixtures thereof.

21. The product according to any of claims 18 to 20, wherein the said hydrophilic treatment is accomplished by immersion of the web structure in a bath having a wetting additives concentration ranging between 1 and 10 g/l, or between 1 and 5 g/l, followed by squeezing and heat drying.

22. The product according to any of claims from 1 to 21, wherein the said substance is selected from:
solutions or lotions for personal hygiene and/or sanitization, skin creams, lotions or waxes, tanning creams or lotions, sunscreen formulations, insect repellent formulations, deodorants, perfumes, antibacterial, antiviral and/or antifungal formulations, make-up removing lotions or solutions and cosmetic products in general;
detergent solutions or emulsions, waxes for ceramic or wooden floor, waxes for wood furniture surfaces, surface disinfectants, antibacterial, antiviral and/or antifungal products for household use, metal polishing emulsions, solutions or creams and cleaning formulations in general;
skin disinfectants, skin antibacterial, antiviral and/or antifungal substances, cicatrizing formulations and in general any drug formulation that can be administered by topic application, or sanitizing compositions for medical facilities, appliances or devices.

23. The product according to claim 22, wherein the substance is a viscous substance selected from a cream, a wax, an emulsion, a suspension or a gel and wherein the said nonwoven fabric is air-through bonded and is made hydrophilic by treatment with disodium cocopolyglucose solphosuccinate.

24. The product according to any of claims from 1 to 23, wherein the said nonwoven fabric has an apparent density, defined as the weight per volume unit, wherein the volume is given by the fibres volume and the voids volume, is between 20 gr/dm³ and 30 gr/dm³.

25. The product according to any of claims from 1 to 24, wherein the fibres or continuous filaments have a diameter between 0.5 dtex and 6.7 dtex; or a diameter of between 0.9 and 2.5 dtex, or about 2 dtex, for the continuous filaments; or a diameter of between 0.5 and 3.7 dtex, or about 3.3 dtex, for the staple fibres.

26. The product according to any of claims 1 to 25, the product being apt to releasing more than 80 vol%, or more than 90 vol% of the substance from the nonwoven fabric product upon pressure application on a surface to be treated.

27. A process for preparing the wet product as described in any of claims 1 to 26, wherein the said bonded web structure is a spunlace, spunlace hydroembossed, dry-laid or chemically bonded web structure, has a content of starting additives of <0,1% to 0,2% and is subjected to i) a hydrophilic treatment with wetting additives by means of a technique selected among spraying, plating, printing, impregnation, application of foam and wherein the concentration of the wetting additives solution ranges between 1 and 10 g/l, or between 1 and 5 g/l, and ii) a step of impregnation with a substance for personal care, household application or topic medical use.

28. The process according to claim 27, comprising a treatment step with an anti-foam additive simultaneously with the wetting treatment.
